# EUROPEAN PATENT SPECIFICATION

(11) **EP 1 848 426 B1**
(45) Date of publication and mention of the grant of the patent: **01.08.2012**
(21) Application number: 06709609.9
(22) Date of filing: 02.02.2006
(51) Int. Cl.: A61K 31/403, A61K 31/407, A61K 31/404, A61K 31/405, A61P 9/10, A61P 25/28, A61P 25/16, A61P 25/14, C07D 209/88, C07D 495/04, C07D 209/94, C07D 491/10

(54) **TRICYCLIC CYTOPROTECTIVE COMPOUNDS**
TRICYCLISCHE ZELLSCHUTZVERBINDUNGEN
COMPOSES CYTOPROTECTEURS TRICYCLIQUES

(30) Priority: 03.02.2005 GB 0502267
(43) Date of publication of application: 31.10.2007
(73) Proprietor: Hunter-Fleming Limited, Bristol BS1 6EA (GB)
(72) Inventor: WÜLFERT, Ernst, B-1180 Brussels (BE); KHALAF, Abedawn Ibrahim, Glasgow G1 1XQ (GB); MACKAY, Simon Paul, Glasgow G1 1XQ (GB); JOHNSTON, Blair Fraser, Glasgow G1 1XQ (GB); SUCKLING, Colin James, Glasgow G1 1XQ (GB)
(74) Representative: Mancini, Vincenzo
(86) International application number: PCT/GB2006/000358
(87) International publication number: WO 2006/082409

(56) References cited:
- EP-A- 0 310 179
- EP-A- 0 358 957
- EP-A- 1 184 373
- WO-A-00/02878
- WO-A-01/87038
- WO-A-95/24191
- WO-A-96/03377
- WO-A-98/04527
- WO-A-2005/009370
- WO-A-2005/026112
- WO-A-2006/011750
- US-A- 5 451 600
- US-B1- 6 350 885
- DATABASE BEILSTEIN [Online] BEILSTEIN INSTITUTE FOR ORGANIC CHEMISTRY, FRANKFURT-MAIN, DE; XP002392311 Database accession no. BRN 1528848 & SATOMURA M. ET AL.: J. ORG. CHEM., vol. 59, no. 26, 1994, pages 8304-7306,
- DATABASE BEILSTEIN [Online] BEILSTEIN INSTITUTE FOR ORGANIC CHEMISTRY, FRANKFURT-MAIN, DE; XP002392312 Database accession no. BRN 1462652 & TEUBER, C.: JUSTUS LIEBIGS ANN. CHEM., vol. 671, 1964, pages 127-133,
- CAUBÈRE C. ET AL.: "Aggregative activation and heterocyclic chemistry I Complex bases promoted arynic cyclisation of imines or enaminoketones; regiochemical synthesis of indoles" TETRAHEDRON, vol. 50, no. 41, 1994, pages 11903-11920, XP000942085
- KUCHEROVA N.F. ET AL.: "Indole derivatives XII. Synthesis of derivatives of the S,S-dioxide of hydrothiopyrano[4,3-b]indole" J. GEN. CHEM. USSR (ENGL. TRANSL.), vol. 33, 1963, pages 3331-3335, XP008066548
- NICOLAUS B J R: "Symbiotic Approach to Drug Design" DECISION MAKING IN DRUG RESEARCH, 1983, pages 173-186, XP002197412
- DATABASE BIOSIS [Online] BIOSCIENCES INFORMATION SERVICE, PHILADELPHIA, PA, US 1994 EHLERT FREDERICK J ET AL: 'Muscarinic receptors and novel strategies for the treatment of age-related brain disorders' Database accession no. PREV199598054577 & LIFE SCIENCES, vol. 55, no. 25-26, 1994, pages 2135-2145, ISSN: 0024-3205
- DATABASE REGISTRY [Online] CHEMICAL ABSTRACTS SERVICE, COLUMBUS, OHIO, US 05 September 2002 Database accession no. [446829-40-9]
- LI CHUANYU ET AL: "Reactive species mechanisms of cellular hypoxia-reoxygenation injury", AMERICAN JOURNAL OF PHYSIOLOGY, vol. 282, no. 2 part 1, February 2002 (2002-02), pages C227-C241, ISSN: 0002-9513

## Description

The present invention relates to the protection against damage caused by ischaemic stress of peripheral organs which are selected from any functional tissue in the body except the brain and the spinal cord, such as the heart or the kidneys, using the bicyclic neuroprotective compounds as defined in the claims.

In tissues affected by low oxygen states, such as prolonged hypoxia and ischaemia, which may or may not be associated with hypoglycaemia, neuronal damage, to varying degrees, is encountered. Ischaemia typically occurs as a result of an acute event, for example heart attack, stroke or traumatic head injury. During heart attack, the damage incurred is substantially limited to the heart tissues, and certain treatments have been developed. In stroke or traumatic head injury, neuronal damage results from the effects of more long term ischaemia on the brain. The severity of the ischaemia depends on the nature of the stroke or injury, but, invariably, there is brain damage. WO99/31049 addresses the effects of ischaemia on the brain such as occurs with stroke patients or as a result of head injury, and discloses certain neuroprotective agents and their use in treating neuronal damage caused by acute ischaemic events such as stroke and head injury.

In contrast to the neuronal damage occurring as a result of acute ischaemic events such as heart attack, stroke or head injury, the underlying causes of chronic neurodegenerative diseases or conditions, such as Alzheimer's Disease (AD), Parkinson's Disease (PD), Huntington's Chorea (HC), Multiple Sclerosis (MS) and Amyotrophic Lateral Sclerosis (ALS), are complex and appear to be multifactorial. In each case, necrotic and apoptotic neuronal cell death may result from one or more mechanisms including metabolic compromise, excitotoxicity and oxidative stress. A number of studies point towards oxidative stress as a major causative factor in a variety of chronic neurodegenerative diseases including AD, PD and ALS (for example, see: Sayre et al., (2001), Curr. Med. Chem, 8(7), 721-38; Bains et al., (1997), Brain Res. Rev., 25, 335-358; Alexi et al. (2000), Progress in Neurobiol., 60, 409-470).

Oxidative stress occurs when the normal balance between oxidative events and antioxidative defence mechanisms is disrupted, either by the loss of reducing agents and/or antioxidants or by increased levels of oxidant species. Oxidative stress has been attributed to the actions of highly toxic free-radicals, including reactive oxide species (ROS) such as the superoxide anion (*O₂⁻) and hydroxyl radical (*OH), and reactive nitrogen species (RNS) derived from nitric oxide (NO) reaction with superoxide or peroxide, such as peroxinitrite (*ONOO⁻).

Excitotoxic cell death is caused by excessive activation of glutamate receptors by glutamate and glutamatergic agonists such as NMDA and other excitatory amino acids (EAAs). A number of studies also suggest that oxidative stress may act as a mediator in excitotoxically induced neuronal cell death. For example, it has been shown for both NMDA and kainate (a non-NMDA receptor agonist) that activation of EAA receptors increases free-radical damage to lipids, and that this damage can be prevented by simultaneous treatment with antioxidants.

Metabolic compromise may be caused by stroke, asphyxiation, hypoglycaemia and certain poisons interfering with mitochondrial respiration. Mitochondrial dysfunction and resulting depletion of ATP and loss of intracellular calcium buffering capacity can cause an increase in the production of reactive oxygen and nitrogen free-radicals, leading to oxidative stress.

Thus, not only is oxidative stress by free-radicals understood to be a primary factor of neuronal cell death in a number of chronic neurodegenerative diseases, but it may also mediate excitotoxic stimuli and metabolic compromise. Furthermore, the reverse interaction may also occur, as oxidative stress by free-radicals may initiate excitotoxic pathways and cause metabolic impairment.

EP-A-0 310 179 and US-A-5 451 600 disclose the use of tetrahydrocarbazoles for the treatment or prevention of cardiac, renal or hepatic ischaemic diseases.

We have now found that certain tricyclic compounds may be used to treat chronic and acute neurodegenerative diseases or conditions and for the prevention or treatment of damage caused by ischaemic stress of peripheral organs, such as the heart or the kidneys.

Thus, the present invention provides the use of compounds of formula (I) and pharmaceutically acceptable salts thereof for the manufacture of a medicament for protection against ischaemic damage to tissues of peripheral organs. The compounds used have the formula (I): 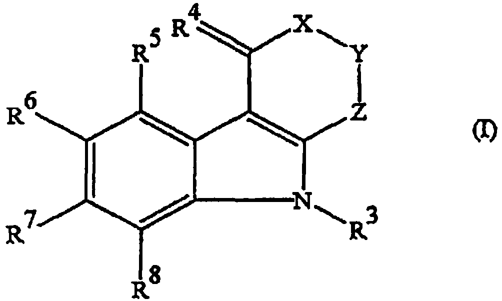 in which:
X represents a group of formula >CR¹R² or, when R⁶ does not represent a hydrogen atom, a group of formula >SO₂;
Y represents a group of formula >CR¹R²;
Z represents a group of formula >C=O, a group of formula >CH₂ or a direct bond;
R¹ represents a hydrogen atom and R² represents a hydrogen atom, a carboxy group or a hydroxy group;
   or
R¹ and R² together represent an oxo group, an ethylenedioxy group or a hydroxyimino group;
R³ represents a hydrogen atom or a straight or branched C₁-C₁₀ alkyl group;
R⁴ represents two hydrogen atoms, or an oxo or hydroxyimino group;
R⁵ represents a hydrogen atom, a straight or branched C₁-C₁₀ alkyl group or a halogen atom;
R⁶ represents a hydrogen atom, a straight or branched C₁-C₁₀ alkoxy group or a carboxy group;
R⁷ and R⁸ are the same as or different from each other and each represents a hydrogen atom, a straight or branched C₁-C₁₀ alkyl group or a halogen atom;
and salts when the compound contains a carboxy group.

Certain of the compounds of formula (I) are new compounds per se.

In the compounds of the present invention, Z may be a direct bond, in which case it forms part of a 5-membered ring fused to a 5-membered nitrogen-containing heterocyclic ring, or it may be a group of formula >CH₂ or >C=O, in which case it forms part of a 6-membered ring.

Where R³, R⁵, R⁷ or R⁸ represent a straight or branched chain alkyl group having from 1 to 10 carbon atoms this may be preferably from 1 to 6 carbon atoms. Examples of such groups include the methyl, ethyl, propyl, isopropyl, butyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl, isohexyl, heptyl, octyl, nonyl and decyl groups, of which the methyl, ethyl, propyl, butyl and hexyl groups are preferred, the methyl and ethyl groups being more preferred, and the methyl group being most preferred.

Where R⁵, R⁷ or R⁸ represents a halogen atom, this may be a fluorine, chlorine, bromine or iodine atom, of which the fluorine and chlorine atoms are preferred.

Where R⁶ represents a straight or branched chain alkoxy group having from 1 to 10 carbon atoms this may be preferably from 1 to 6 carbon atoms. Examples of such groups include the methoxy, ethoxy, propoxy, isopropoxy, butoxy, sec-butoxy, t-butoxy, pentyloxy, isopentyloxy, neopentyloxy, 2-methylbutoxy, 1-ethylpropoxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, 1,2-dimethylbutoxy, 1,3-dimethylbutoxy, 2,3-dimethylbutoxy, 2-ethylbutoxy, hexyloxy, isohexyloxy, heptyloxy, octyloxy, nonyloxy and decyloxy groups, of which the methoxy, ethoxy, propoxy, butoxy and hexyloxy groups are preferred, the methoxy and ethoxy groups being more preferred, and the methoxy group being most preferred.

Of the compounds of the present invention, we particularly prefer those in which:
X represents a group of formula >CR¹R² where R¹ represents a hydrogen atom and R² represents a hydrogen atom, a hydroxy group or a carboxy group, or R¹ and R² together represent an oxo group or an ethylenedioxy group;
Y represents a group of formula >CR¹R² where R¹ represents a hydrogen atom and R² represents a hydrogen atom or a carboxy group;
R³ represents a hydrogen atom;
R⁴ represents two hydrogen atoms or an oxo group;
R⁵ represents a hydrogen atom;
R⁶ represents a hydrogen atom, a C₁ - C₄ alkoxy group or a carboxy group; and R⁷ and R⁸ are the same as or different from each other and each represents a hydrogen atom or a C₁ - C₄ alkyl group;
and salts thereof.

Specific examples of compounds of the present invention are given in the following Table 1:

**Table 1**

| | |
|---|---|
| 1 | 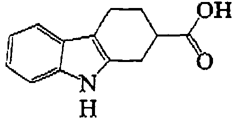 |
| 2 | 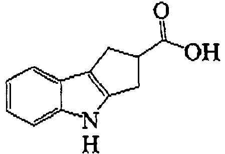 |
| 3 | 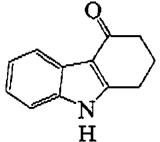 |
| 4 | 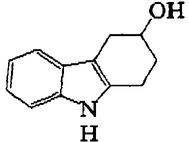 |
| 6 | 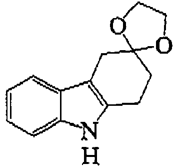 |
| 7 | 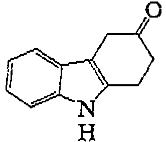 |
| 8 | 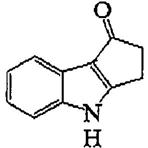 |
| 9 | 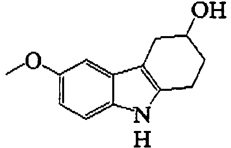 |
| 10* | 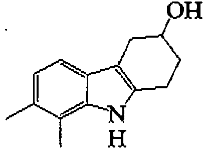 |
| 11* | 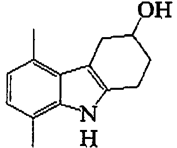 |
| 12* | 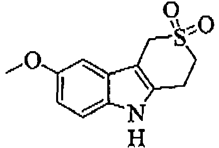 |
| 13 | 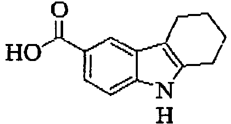 |
| 14 | 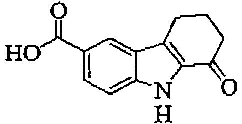 |
| 15 | 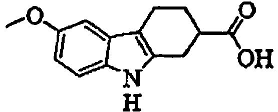 |
| 16 | 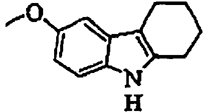 |
| 17 | 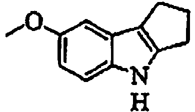 |
| 18 | 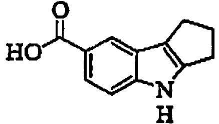 |
| 19* | 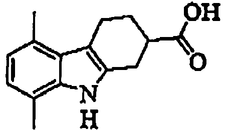 |
| 20 | 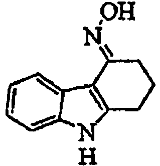 |
| 21 | 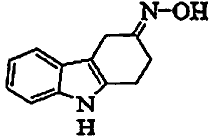 |
| 22 | 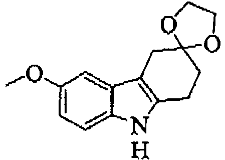 |
| 23 | 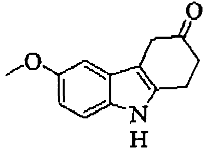 |

In the above Table 1, those compounds marked with an asterisk are per se new compounds and also form a part of the present invention. The most preferred compounds of the present invention are those compounds numbered 4, 6, 7, 8, 10, 14, 15, 16, 17, 20, 21, 22 and 23 in the above Table.

When the compounds of the present invention contain a carboxy group, for example when R¹ or R⁶ represents a carboxy group, the compounds of the invention can form esters, which may be prepared by conventional esterification techniques. There is no particular restriction on the nature of the ester, provided that, where the resulting compound is to be used medically, the compound is pharmaceutically acceptable, that is it is not less active, or unacceptably less active, nor more toxic, or unacceptably more toxic, than the parent compound. However, where the compound is to be used for non-medical uses, e.g. as an intermediate in the preparation of other compounds, even this restriction does not apply, and there is then no restriction on the nature of the esters which may be formed.

Examples of ester groups include:
alkyl groups having from 1 to 20 carbon atoms, more preferably from 1 to 10 carbon atoms, such as those exemplified in relation to R³, R⁵, R⁷ or R⁸ and higher alkyl groups as are well known in the art, such as the dodecyl, tridecyl, pentadecyl, octadecyl, nonadecyl and icosyl groups;
cycloalkyl groups having from 3 to 7 carbon atoms, for example the cyclopropyl, cyclobutyl, cyclopentyl, cyclohexyl and cycloheptyl groups;
aralkyl groups, in which the alkyl part has from 1 to 3 carbon atoms and the aryl part is a carbocyclic aromatic group having from 6 to 14 carbon atoms, which may be substituted or unsubstituted; examples of such aralkyl groups include the benzyl, phenethyl, 1-phenylethyl, 3-phenylpropyl, 2-phenylpropyl, 1-naphthylmethyl, 2-naphthylmethyl, 2-(1-naphthyl)ethyl, 2-(2-naphthyl)ethyl, benzhydryl (i.e. diphenylmethyl), triphenylmethyl, bis(o-nitrophenyl)methyl, 9-anthrylmethyl, 2,4,6-trimethylbenzyl, 4-bromobenzyl, 2-nitrobenzyl, 4-nitrobenzyl, 3-nitrobenzyl, 4-methoxybenzyl and piperonyl groups;
alkenyl groups having from 2 to 6 carbon atoms, such as the vinyl, allyl, 2-methylallyl, 1-propenyl and isopropenyl groups;
halogenated alkyl groups having from 1 to 6, preferably from 1 to 4, carbon atoms, such as the 2,2,2-trichloroethyl, 2-haloethyl (e.g. 2-chloroethyl, 2-fluoroethyl, 2-bromoethyl or 2-iodoethyl), 2,2-dibromoethyl and 2,2,2-tribromoethyl groups;
substituted silylalkyl groups, for example the 2-tri(C₁ - C₄)akylsilylethyl groups, especially a 2-trimethylsilylethyl group;
substituted and unsubstituted phenyl groups, for example the phenyl, tolyl and benzamidophenyl groups;
substituted and unsubstituted phenacyl groups, for example the phenacyl group itself or the p-bromophenacyl group;
cyclic and acyclic terpenyl groups, for example the geranyl, neryl, linalyl, phytyl, menthyl (especially m- and p- menthyl), thujyl, caryl, pinanyl, bornyl, norcaryl, norpinanyl, norbornyl, menthenyl, camphenyl and norbornenyl groups;
alkoxymethyl groups, in which the alkoxy part has from 1 to 6, preferably from 1 to 4, carbon atoms and may itself be substituted by a single unsubstituted alkoxy group, such as the methoxymethyl, ethoxymethyl, propoxymethyl, isopropoxymethyl, butoxymethyl and methoxyethoxymethyl groups;
aliphatic acyloxyalkyl groups, in which the acyl group is preferably an alkanoyl group and is more preferably an alkanoyl group having from 2 to 6 carbon atoms, and the alkyl part has from 1 to 6, and preferably from 1 to 4, carbon atoms such as the acetoxymethyl, propionyloxymethyl, butyryloxymethyl, isobutyryloxymethyl, pivaloyloxymethyl, 1-pivaloyloxyethyl, 1-acetoxyethyl, 1-isobutyryloxyethyl, 1-pivaloyloxypropyl, 2-methyl-1-pivaloyloxypropyl, 2-pivaloyloxypropyl, 1-isobutyryloxyethyl, 1-isobutyryloxypropyl, 1-acetoxypropyl, 1-acetoxy-2-methylpropyl, 1-propionyloxyethyl, 1-propionyloxypropyl, 2-acetoxypropyl and 1-butyryloxyethyl groups;
cycloalkyl-substituted aliphatic acyloxyalkyl groups, in which the acyl group is preferably an alkanoyl group and is more preferably an alkanoyl group having from 2 to 6 carbon atoms, the cycloalkyl substituent has from 3 to 7 carbon atoms, and the alkyl part has from 1 to 6, preferably from 1 to 4, carbon atoms, such as the cyclohexylacetoxymethyl, 1-(cyclohexylacetoxy)ethyl, 1-(cyclohexylacetoxy)propyl, 2-methyl-1-(cyclohexylacetoxy)propyl, cyclopentylacetoxymethyl, 1-(cyclopentylacetoxy)ethyl, 1-(cyclopentylacetoxy)propyl and 2-methyl-1-(cyclopentylacetoxy)-propyl, groups;
alkoxycarbonyloxyalkyl groups, especially 1-(allcoxycarbonyloxy)ethyl groups, such as the 1-methoxycarbonyloxyethyl, 1-ethoxycarbonytoxyethyl, 1-propoxycarbonyloxyethyl, 1-isopropoxycarbonyloxyethyl, 1-butoxycarbonyloxyethyl, 1-isobutoxycarbonyloxyethyl, 1-sec-butoxycarbonyloxyethyl, 1-t-butoxycarbonyloxyethyl, 1-(1-ethylpropoxycarbonyloxy)ethyl and 1-(1,1-dipropylbutoxycarbonyloxy)ethyl groups, and other alkoxycarbonylalkyl groups, in which both the alkoxy and alkyl groups have from 1 to 6, preferably from 1 to 4, carbon atoms, such as the 2-methyl-1-(isopropoxycarbonyloxy)propyl, 2-(isopropoxycarbonyloxy)propyl, isopropoxycarbonyloxymethyl, t-butoxycarbonyloxymethyl, methoxycarbonyloxymethyl and ethoxycarbonyloxymethyl groups;
cycloalkylcarbonyloxyalkyl and cycloalkyloxycarbonyloxyalkyl groups, for example the 1-methylcyclohexylcarbonyloxymethyl, 1-methylcyclohexyloxycarbonyloxymethyl, cyclopentyloxycarbonyloxymethyl, cyclopentylcarbonyloxymethyl, 1-(cyclohexyloxycarbonyloxy)ethyl, 1-(cyclohexylcarbonyloxy)ethyl, 1-(cyclopentyloxycarbonyloxy)-ethyl, 1-(cyclopentylcarbonyloxy)ethyl, 1-(cycloheptyloxycarbonyloxy)ethyl, 1-(cycloheptylcarbonyloxy)ethyl, 1-methylcyclopentylcarbonyloxymethyl, 1-methylcyclopentyloxycarbonyloxymethyl, 2-methyl-l-(1-methylcyclohexylcarbonyloxy)-propyl, 1-(1-methylcyclohexylcarbonyloxy)propyl, 2-(1-methylcyclohexylcarbonyloxy)propyl, 1-(cyclohexylcarbonyloxy)propyl, 2-(cyclohexylcarbonyloxy)propyl, 2-methyl-1-(1- methylcyclopentylcarbonyloxy)propyl, 1-(1-methylcyclopentylcarbonyloxy)propyl, 2-(1-methylcyclopentylcarbonyloxy)propyl, 1-(cyclopentylcarbonyloxy)propyl, 2-(cyclopentylcarbonyloxy)propyl, 1-(1-methylcyclopentylcarbonyloxy)ethyl, 1-(1-methylcyclopentylcarbonyloxy)propyl, adamantyloxycarbonyloxymethyl, adamantylcarbonyloxymethyl, 1-adamantyloxycarbonyloxyethyl and 1-adamantylcarbonyloxyethyl groups;
cycloalkylalkoxycarbonyloxyalkyl groups, for example the cyclopropyhnethoxycarbonyloxymethyl, cyclobutylmethoxycarbonyloxymethyl, cyclopentylmethoxycarbonyloxymethyl, cyclohexylmethoxycarbonyloxymethyl, 1-(cyclopropylmethoxycarbonyloxy)ethyl, 1-(cyclobutylmethoxycarbonyloxy)ethyl, 1-(cyclopentylmethoxycarbonyloxy)ethyl and 1-(cyclohexylmethoxycarbonyloxy)ethyl groups;
terpenylcarbonyloxyalkyl and terpenyloxycarbonyloxyalkyl groups, for example the 1-(menthyloxycarbonyloxy)ethyl, 1-(menthylcarbonyloxy)ethyl, menthyloxycarbonyloxymethyl, menthylcarbonyloxymethyl, 1-(3-pinanyloxycarbonyloxy)ethyl, 1-(3-pinanylcarbonyloxy)ethyl, 3-pinanyloxycarbonyloxymethyl and 3-pinanylcarbonyloxymethyl groups;
5-alkyl- or 5-phenyl- (2-oxo-1,3-dioxolen-4-yl)alkyl groups, for example the (5-methyl-2-oxo-1,3-dioxolen-4-yl)methyl, (5-phenyl-2-oxo-1,3-dioxolen-4-yl)methyl, (5-isopropyl-2-oxo-1,3-dioxolen-4-yl)methyl, (5-t-butyl-2-oxo-1,3-dioxolen-4-yl)methyl and 1-(5-methyl-2-oxo-1,3-dioxolen-4-yl)ethyl groups; and
other groups, especially groups which are easily removed in vivo such as the phthalidyl, indanyl and 2-oxo-4,5,6,7-tetrahydro-1,3-benzodioxolen-4-yl groups.

Also, if the compounds of the present invention contain a carboxy group, they can be converted into salts with a base by conventional methods. There is no particular restriction on the nature of such salts, provided that, where the compounds are to be used medically, the compounds are pharmaceutically acceptable. However, where the compound is to be used for non-medical uses, e.g. as an intermediate in the preparation of other compounds, even this restriction does not apply, and there is then no restriction on the nature of the salts which may be formed. Examples of such salts include: salts with an alkali metal, such as sodium, potassium or lithium; salts with an alkaline earth metal, such as barium or calcium; salts with another metal, such as magnesium or aluminium; ammonium salts; organic base salts, such as a salt with methylamine, dimethylamine, triethylamine, diisopropylamine, cyclohexylamine or dicyclohexylamine; and salts with a basic amino acid, such as lysine or arginine. We prefer the pharmaceutically acceptable salts.

The compounds of the present invention can also be converted to salts with acids by conventional methods. There is no particular restriction on the nature of such salts, provided that, where the compounds are to be used medically, the compounds are pharmaceutically acceptable. However, where the compound is to be used for non-medical uses, e.g. as an intermediate in the preparation of other compounds, even this restriction does not apply, and there is then no restriction on the nature of the salts which may be formed. Examples of such salts include: salts with mineral acids, especially hydrohalic acids (such as hydrofluoric acid, hydrobromic acid, hydroiodic acid or hydrochloric acid), nitric acid, perchloric acid, carbonic acid, sulphuric acid or phosphoric acid; salts with lower alkylsulphonic acids, such as methanesulphonic acid, trifluoromethanesulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid, benzoic acid, mandelic acid, ascorbic acid, lactic acid, gluconic acid or citric acid; and salts with amino acids, such as glutamic acid or aspartic acid. We prefer the pharmaceutically acceptable salts.

The compounds used in the present invention are either known or may be prepared by methods analogous to those used to prepare the known compounds.

The compounds of the present invention may therefore be used in the treatment or prophylaxis of chronic and acute neurodegenerative diseases or conditions or for protection against ischaemic damage to tissues of peripheral organs, and, for these purposes, may be formulated as conventional pharmaceutical preparations, as is well known in the art. Thus, the compounds may be administered orally, e.g. in the form of tablets, capsules, granules, powders, syrups, sprays or other such well known forms, or parenterally, e.g. by injections, sprays, eye drops, adhesive plasters or suppositories, etc.

These pharmaceutical preparations can be prepared by conventional means and may contain known adjuvants of a type commonly used in this field, for example vehicles, binders, disintegrators, lubricants, stabilizers, corrigents, etc. depending upon the intended use and form of the preparation. The dose will depend upon the condition, age, and body weight of the patient as well as upon the nature and severity of the disorder to be treated, but in the case of oral administration to an adult human patient, we would normally suggest a total daily dose of from 0.01 to 50 mg/kg body weight (more preferably from 0.05 to 20 mg/kg body weight), which may be administered in a single dose or in divided doses, e.g. from one to three times a day.

The invention is further illustrated by the following Examples, of which Examples 1-23 illustrate the preparation of the compounds, while Example 24 illustrates their therapeutic properties. In the Examples, the following abbreviations are used: br, broad; s, singlet; d, doublet; t, triplet; q, quartet; exch, exchangeable; DCM, dichloromethane; DMF, *N,N*-dimethylformamide; HREIMS, high resolution electron impact mass spectroscopy; HRFABMS, high resolution fast atom bombardment mass spectroscopy; LRESMS, low resolution electrospray mass spectroscopy; NMM, *N-*methylmorpholine; Pd/C, palladium on carbon; mp, melting point; TFA, trifluoroacetic acid, THF, tetrahydrofuran; TLC, thin layer chromatography; SFM, serum free medium; MEM, Minimum Essential Medium with Earle's salt; DMSO, dimethyl sulphoxide. HREIMS and HRFABMS were obtained on a Jeol^{®} JMS-AX505HA mass spectrometer. LRESMS were obtained on a Fisons^{®} VG Platform Benchtop LC-MS. NMR spectra were obtained on a Bruker^{®} AMX 400 spectrometer. IR spectra were run as KBr discs and liquids as films, using a Nicolet^{®} Impact 400D. Column chromatography was performed with silica gel Prolabo^{®} (200-400 mash). The Compound Nos. referred to in the Examples are those assigned to the compounds in the foregoing Table 1.

### EXAMPLE 1

### 2,3,4,9-Tetrahydro-1H-carbazole-2-carboxylic acid [Compound No. 1]

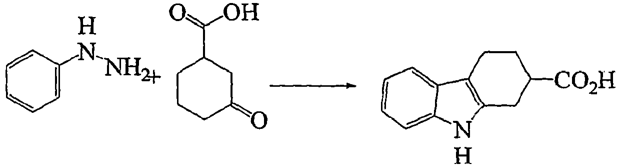

3-Oxocyclohexanecarboxylic acid (200 mg, 1.407 mmol) was dissolved in acetic acid (3 mL) to which a solution of phenylhydrazine (160 mg, 1.480 mmol) in acetic acid (2 mL) was added at room temperature, with stirring. The reaction mixture was heated until reflux for 2 hours. The reaction mixture was then cooled to room temperature, diluted with ethyl acetate and extracted with brine. The organic layers were collected and dried (Na₂SO₄), and the solvent was removed under reduced pressure. The crude product was re-crystallised from ethyl acetate/n-hexane to give a light brown microcrystalline solid (191 mg, 63%), m.p.235-238°C [c.f. Asselin, A. A., et al, J. Med. Chem., 1976, 19, 787-792, m.p.239-241°C; Allen, et al, J. Heterocyclic Chem., 1970, 7, 239-241, m.p.233-235°C]. IR (KBr): 3416, 3048, 2923, 2844, 1689, 1465, 1444, 1415, 1288, 1264, 1226, 935, 740 cm.⁻¹¹H NMR (DMSO-d₆): δ 12.27(1H, s), 10.66(1H, s), 7.33(1H, d, J=7.6Hz), 7.24(1H, d, J=7.6Hz), 6.98(1H, dt, J=1.2Hz and J=6.8Hz), 6.91(1H, dt, J=1.2Hz and J=6.8Hz), 2.89-2.62(5H, m), 2.17(1H, m), 1.87-1.81(1H, m).

### EXAMPLE 2

### 1,2,3,4-Tetrahydrocyclopenta[b]indole-2-carboxylic acid [Compound No. 2]:

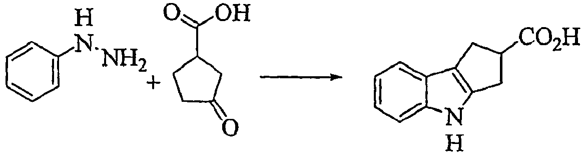

3-Oxocyclopentanecarboxylic acid (200 mg, 1.561 mmol) was dissolved in acetic acid (3 mL) to which phenylhydrazine (180 mg, 1.665 mmol) in acetic acid (2 mL) was added at room temperature, with stirring. The reaction mixture was heated until reflux for 2 hours. It was then left to cool to room temperature, diluted with ethyl acetate and extracted with brine. The organic layers were collected and dried (Na₂SO₄), and the solvent was removed under reduced pressure to give a black mass, which was applied to a silica gel column chromatography using 1/1 ethyl acetate/hexane to elute the product. Fractions containing the required material (R_{F}=0.50) were collected and the solvents were removed under reduced pressure. The product was re-crystallised from ethyl acetate/hexane to give a light brown microcrystalline solid (48 mg, 15%), m.p.215-217°C [c.f. Lacoume, B.; Milcent, G. and Olivier, A., Tetrahedron, 1972, 28, 667-674.m.p.215°C]. IR (KBr): 3362, 3028, 2910, 2859, 1691, 1438, 1411, 1323, 1277, 1238, 741 cm.^{-1 1}H NMR(DMSO-d₆): 12.27(1H, s), 10.81(1H, s), 7.31(1H, d, J=7.6Hz), 7.27(1H, d, J=7. 6Hz), 6.97(1H, dt, J=1.2Hz and J=6.8Hz), 6.92(1H, dt, J=1.2Hz and J=6.8Hz), 3.76-3.68(1H, m), 3.09-3.03(3H, m), 2.95(1H, m).

### EXAMPLE 3

### 1,2,3,9-Tetrahydro-4H-carbazol-4-one [Compound No. 3]

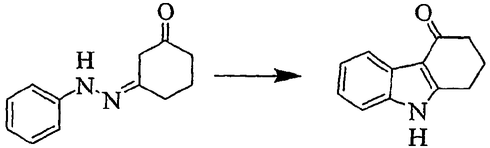

(1*E*)-1,3-Cyclohexanedione 1-(phenylhydrazone) (2.031 g, 10.045 mmol) was dissolved in TFA (10 mL) at room temperature, with stirring. The reaction mixture was heated under reflux for 8 hours, after which it was left at room temperature overnight. The resulting dark-coloured solution was poured slowly over ice water, with stirring. The semi-solid material was extracted with ethyl acetate and dried (MgSO₄), and the solvent was removed under reduced pressure, to give pale yellow solid which, upon recrystallisation from methanol, gave 1.004 g. The mother-liquor was concentrated and applied to a silica gel column chromatography using ethyl acetate/n-hexane (1/1) R_{F}=0.15, to give an additional amount of the product as a white solid (80 mg) after the recrystallisation from methanol. The total weight of the product obtained was 1.084 g, 58%, mp226-228°C [Clemo, G.R. and Felton, D.G.I, J.Chem.Soc., 1951, 700-702, mp223°C]. IR (KBr): 3144, 2937, 1622, 1581, 1468, 1406, 1250, 1175, 1140, 751 cm.^{-1 1}H NMR (DMSO-d₆): 11.83(1H, br); 7.95(1H, dd, J=2.3Hz & J=6.7Hz); 7.39(1H, dd, J=1.3Hz & J=6.3Hz); 7.18-7.11(2H, m); 2.96(2H, t, J=6.1Hz); 2.42(2H, t, J=6.1Hz); 2.12(2H, q, J=6.4Hz).

### EXAMPLE 4

### 2,3,4,9-Tetrahydro-1H-carbazol-3-ol [Compound No. 4]:

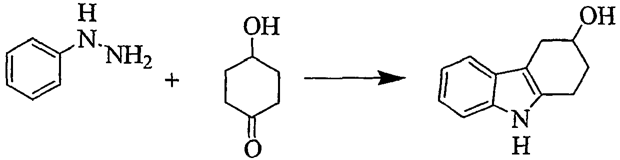

To a solution of 4-hydroxycyclohexanone (0.970 g, 8.220 mmol) in acetic acid (10 mL) phenylhydrazine (1.216 g, 11.249 mmol) was added dropwise, with stirring, at room temperature. The material began to crystallise almost immediately and additional acetic acid (5 mL) and ethanol (5 mL) were added. The reaction mixture was then heated under reflux for 3 hours. The resulting dark red solution was concentrated under reduced pressure to about 6 mL and then diluted with a sufficient amount of water to produce clouding. Cooling and scratching induced crystallisation. The mixture was filtered and the solid was washed with water. Crystallisation from methanol/water followed by recrystallisation from ethyl acetate/n-hexane gave the required product as a tan solid (670 mg, 44%), mp148-150°C [Gardner, P.D.; Haynes, G.R. and Brandon, R.L., J.Org.Chem., 1957, 22, 1206-1210, mp148.5-149.5°C], R_{F}=0.32 (ethyl acetate/n-hexane 1/1). IR(KBr): 3384, 2920, 2843, 1620, 1453, i367, 1324, 1054, 1004, 744, 637 cm.⁻¹ H¹ NMR (CDCl₃): 7.81 (1H, br); 7.52(1H, d, J=7.4Hz); 7.34(1H, d, J=7.4Hz); 7.19(1H, t, J=6.3Hz); 7.16(1H, t, 6.3Hz); 4.33(1H, m); 3.18(1H, dd, J=4.8Hz & J=15.2Hz); 2.98-2.74(3H, m); 2.22-2.04(2H, m); 1.77(1H, br).

### EXAMPLE 6

### (a) 1,4-Dioxaspiro[4.5]decan-8-one phenylhydrazine

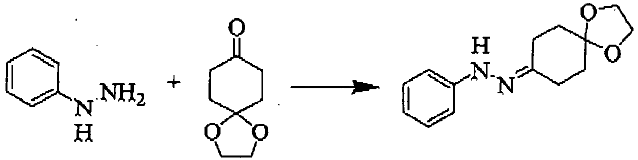

Phenylhydrazine (1.098 g, 10.155 mmol) was dissolved in toluene (20 mL), to which was added 1,4-dioxaspiro[4.5]decan-8-one (1.586 g, 10.155 mmol). The reaction mixture was then heated under reflux for 30 minutes, after which the solvent was removed under reduced pressure, to give the product as an orange oil (2.370 g, 95%), which was used in the next reaction without further purification.

### (b) 1,2,4,9-Tetrahydo-spiro[carbazole-3,2'-[1,3-dioxolane] [Compound No. 6]

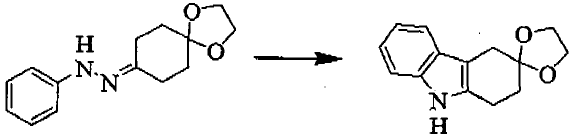

1,4-Dioxaspiro[4.5]decan-8-one phenylhydrazine [2.360 g, 9.581 mmol, prepared in step (a)] was dissolved in ethylene glycol (25 mL). The reaction mixture was heated at 180°C for 4 hours and then left to cool to room temperature, after which it was poured over water at 0°C, extracted with dichloromethane (50 mL) and dried (MgSO₄). The product was obtained as a pink solid, which was recrystallised from ethyl acetate/n-hexane to give a white solid (1.030 g, 47%), R_{F}=0.75 (ethyl acetate/n-hexane 1/1), mp146-148°C [Urrutia, A. and Rodriguez, J.G., Tetrahedron, 1999, 55, 11095-11108, mp146-148°C]. IR (KBr): 3405, 2975, 2897, 1620, 1587, 1463, 1436, 1373, 1296, 1152, 1097, 1054, 1019, 945, 739 cm.^{-1 1}H NMR (CDCl₃): 7.75(1H, br); 7.43(1H, d, J=7.5Hz); 7.28(1H, d, J=7.5); 7.12(1H, dt, J=6.0Hz & 1.3Hz); 7.07(1H, dt, J=6.0Hz & 1.3Hz); 4.11-4.03(4H, m); 2.98(2H, s); 2.95(2H, t, J=6.5Hz); 2.09(2H, t, J=6.5Hz).

### EXAMPLE 7

### 1,2,4,9-Tetrahydro-3H-carbazol-3-one [Compound No. 7]

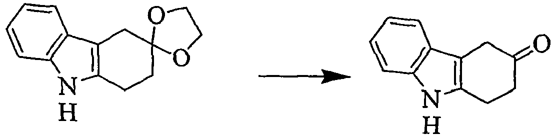

To a solution of 1,2,4,9-Tetrahydo-spiro[carbazole-3,2'-[1,3-dioxolane] (200 mg, 8.723 mmol) in THF (30 mL), was added hydrochloric acid (7 mL, 15%). The mixture was stirred at room temperature for 2 hours, neutralised with sodium carbonate (solid), and then extracted with dichloromethane and dried (MgSO₄). The solvent was removed under reduced pressure and the crude product was purified by column chromatography using ethyl acetate/n-hexane (1/2) as eluant. The product was obtained as a white microcrystalline solid (120 mg, 65%), R_{F}=0.17, mp156-158°C [Urrutia, A. and Rodriguez, J.G., Tetrahedron, 1999, 55, 1.1095-11108, mp157-159°C]. IR (KBr): 3382, 2962, 2915, 1707, 1465, 1435, 1328, 1164, 990, 745 cm.^{-1 1}H NMR (CDCl₃): 7.88(1H, br); 7.45(1H, d, J=7.7Hz); 7.35(1H, d, J=7.7Hz); 7.19(1H, t, J=7.1Hz); 7.13(1H, t, J=7.1Hz); 3.63(2H, s); 3.19(2H, t, J=6.9Hz); 2.82(2H, t, J=6.9Hz).

### EXAMPLE 8

### 3,4-dihydrocyclopenta[b]indol-1(2H)-one [Compound No. 8]

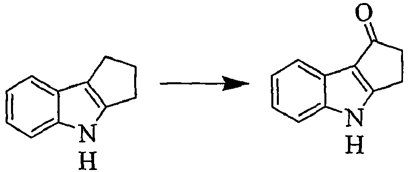

An ice-cooled solution of 1,2,3,4-tetrahydrocyclopenta[*b*]indole (1.006 gm, 6.361 mmol) in a mixture of THF (15 mL) and water (1.5 mL) was deoxygenated by passing a stream of nitrogen through it for 10 minutes. It was then maintained under an atmosphere of nitrogen whilst a solution of 2,3-dichloro-5,6-dicyano-1,4-benzoquinone (3.206 g, 14.123 mmol) dissolved in THF (12 mL) was added dropwise over a period of 10 minutes. Stirring was then continued for a further hour, the reaction mixture being allowed to warm to room temperature during this period. Evaporation of the solvent then left a red-brown solid residue, which was applied to a chromatography column and the products eluted using ethyl acetate only. Three columns were used to purify this material. The product was obtained as a yellow solid (300 mg, 28%), mp252-255°C (decomposition), R_{F}=0.10 (ethyl acetate/n-hexane 1/1), [Rodriguez, J.G.; Temprano, F.; Esteban-Calderon, C.; Martinez-Ripoll, M.; Garcia-Blanco, S., Tetrahedron, 1985, 41(18), 3813-3823, mp257-259°C]. IR(KBr): 3210, 1655, 1614, 1471, 1429, 1241, 1152, 1047, 738 cm.^{-1 1}H NMR (DMSO-d₆): 12.01(1H, br); 7.67(1H, d, J=7.3Hz); 7.45(1H, d, J=7.3Hz); 7.21(1H, dt, J=7.2Hz & 1.4Hz); 7.15(1H, dt, J=7.2Hz & 1.4Hz); 3.08(2H, m); 2.82(2H, m).

### EXAMPLE 9

### 6-Methoxy-2,3,4,9-tetrahydro-1H-carbazol-3-ol [Compound No. 9]

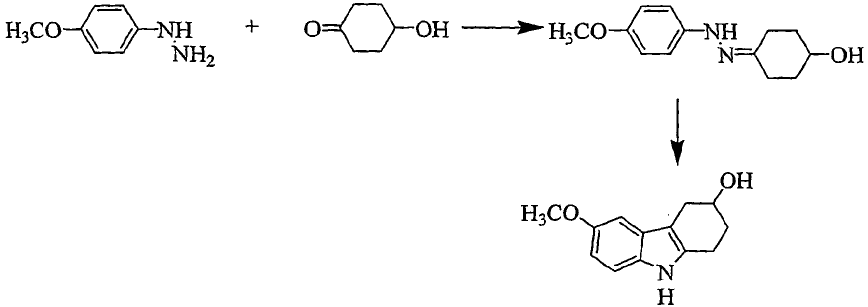

4-Methoxyphenylhydrazine (0.644 g, 3.688 mmol) was suspended in acetic acid (20 mL) and ethanol (10 mL). 4-Hydroxycyclohexanone (0.420 mg, 3.688 mmol) was dissolved in acetic acid (10 mL) and then added to the reaction mixture at room temperature, with stirring. The reaction mixture was heated under reflux for 3 hours. The solvents were partially removed under reduced pressure and then the reaction mixture was diluted with water (25 mL) and extracted with ethyl acetate (2x50 mL). The organic layers were collected and dried (MgSO₄), and the solvents were removed under reduced pressure to give a brown oil. The crude product was purified by column chromatography using silica gel and (ethyl acetate/n-hexane ½). Fractions containing the required material were collected and the solvent removed under reduced pressure to give the product as a fine crystalline material (661 mg, 83%), mp100-103°C [C.W. Bird, A.G.H. Wee, J.Heterocycl.Chem., 1985, 22, 191-192, mp103-106°C], R_{F}=0.17 (ethyl acetate/n-hexane 1/1). IR (KBr): 3392, 2916, 2841, 1622, 1590, 1483, 1436, 1214, 1176, 1050, 1020, 830, 798 cm.^{-1 1}H NMR (CDCl₃): 7.60(1H, br), 7.19(1H, d, J=8.7Hz), 6.92(1H, d, J=2.4Hz), 6.81(1H, dd, J=2.4 & 8.71Hz), 4.29(1H, m), 3.86(3H, s), 3.09-2.67(4H, m), 2.11-2.02(2H, m), 1.75(1H, br).

### EXAMPLE 10

### 7,8-Dimethyl-2,3,4,9-tetrahydro-1H-carbazol-3-ol[Compound No. 101

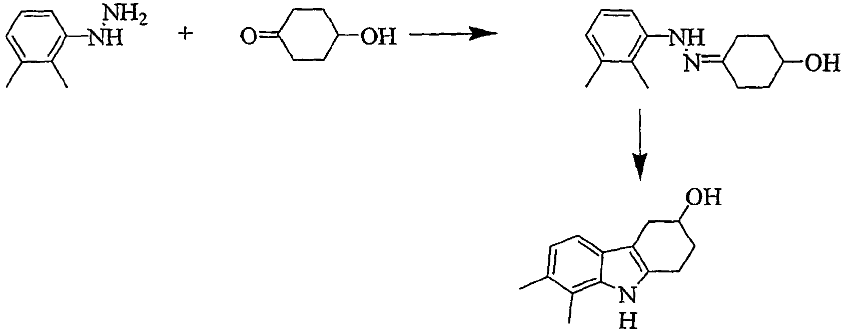

2,3-Dimethylphenylhydrazine (0.643 g, 3.723 mmol) was suspended in acetic acid (20 mL) and ethanol (10 mL). 4-Hydroxycyclohexanone (425 mg, 3.723 mmol) was dissolved in acetic acid (10 mL) and then added to the reaction mixture at room temperature, with stirring. The reaction mixture was then heated under reflux for 3 hours. The solvents were partially removed under reduced pressure and then the reaction mixture was diluted with water (25 mL) and extracted with ethyl acetate (2x50 mL). The organic layers were collected and dried (MgSO₄), and the solvents were removed under reduced pressure to give a brown oil. The crude product was purified by column chromatography using silica gel and (ethyl acetate/n-hexane ½). Fractions containing the required material were collected and the solvent was removed under reduced pressure. The product was obtained as a pale yellow microcrystalline material (410 mg, 51%), mp192-195°C, R_{F}=0.39 (ethyl acetate/n-hexane 1/1). IR (KBr): 3408, 2915, 2847, 1622, 1592, 1443, 1413, 1365, 1324, 1086, 1037, 795 cm.^{-1 1}H NMR (DMSO-d₆): 10.32(1H, s), 7.02(1H, d, J=7.8Hz), 6.73(1H, d, J=7.8Hz), 4.72(1H, d, J=4.3Hz), 3.94(1H, m), 2.87-2.67(3H, m), 2.43(1H, m), 2.30(3H, s), 2.27(3H, s), 1.97(1H, m), 1.78-1.69(1H, m). HRFABMS: Found 216.13952 calculated for C₁₄H₁₈ON 216.13884.

### EXAMPLE 11

### 5,8-Dimethyl-2,3,4,9-tetrahydro-1H-carbazol-3-ol [Compound No. 11]

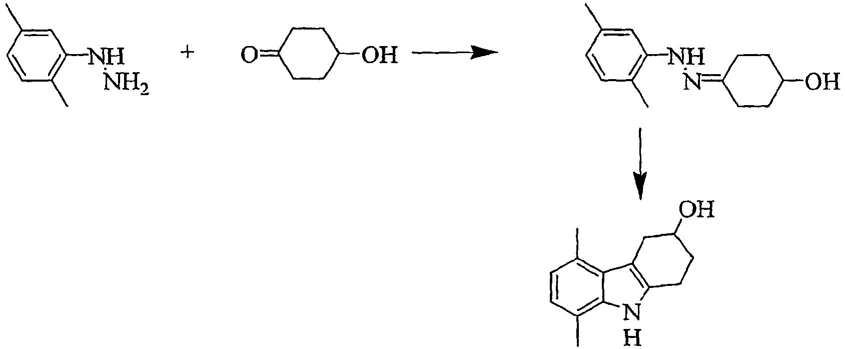

2,5-Dimethylphenylhydrazine (0.643 g, 3.723 mmol) was suspended in acetic acid (20 mL) and ethanol (10 mL). 4-Hydroxycyclohexanone (0.425 mg, 3.723 mmol) was dissolved in acetic acid (10 mL) and then added to the reaction mixture at room temperature, with stirring. The reaction mixture was then heated under reflux for 3 hours. The solvents were partially removed under reduced pressure and then the reaction mixture was diluted with water (25 mL) and extracted with ethyl acetate (2x50 mL). The organic layers were collected and dried (MgSO₄), and the solvents were removed under reduced pressure to give a brown oil. The crude product was purified by column chromatography using silica gel and (ethyl acetate/n-hexane ½). Fractions containing the required material were collected and the solvent was removed under reduced pressure. The product was obtained as a pale yellow microcrystalline material (431 mg, 54%), mp158-161°C, R_{F}=0.39 (ethyl acetate/n-hexane 1/1). IR (KBr): 3423, 3269, 2930, 2852, 1615, 1580, 1514, 1456, 1378, 1327, 1054, 1030, 801 cm.^{-1 1}H NMR (DMSO-d₆): 1041(1H, s), 6.61(1H, d, J=7.3Hz), 6.52(1H, d, J=7.3Hz), 4.72(1H,d, J=4.2Hz), 3.93(1H, m), 3.23(1H, m), 2.76-2.67(3H, m), 2.51(3H, s), 2.32(3H, s), 1.98(1H, m), 1.73(1H, m). HRFABMS: Found 216.13783 calculated for C₁₄H₁₈ON 216.13884.

### EXAMPLE 12

### 8-Methoxy-1,3,4,5-tetrahydrothiopyrano[4,3-b]indole 2,2-dioxide [Compound No. 12]

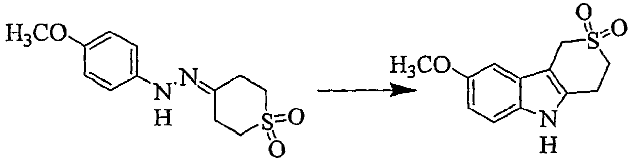

4-Methoxyhydrazine hydrochloride (1.228 g, 7.031 mmol) and tetrahydro-4*H-*thiopyran-4-one 1,1-dioxide (1.042 g, 7.031 mmol) were suspended in ethanol (25 mL). The reaction mixture was then heated under reflux for 1 hour. The solid material that precipitated was filtered off, washed with water and a small amount of ethanol and then dried under reduced pressure at 60°C to give the desired material as a brown solid (789 mg, 45%), mp283-286°C (decomposition). IR (KBr): 3343, 2994, 2937, 1623, 1592, 1484, 1455, 1314, 1269, 1219, 1164, 1101, 1024, 892, 814 cm.^{-1 1}H NMR (DMSO-d₆): 10.93(1H, s), 7.21(1H, d, J=8.7Hz), 6.95(1H, d, J=2.3Hz), 6.73(1H, dd, J=2.4 & 8.7Hz), 4.38(2H,s), 3.74(3H, s), 3.45(2H, t, J=6.1Hz), 3.23(2H, t, J=6.1Hz). HRFABMS: Found 252.06877 calculated for C₁₂H₁₄O₃NS 252.06944.

### EXAMPLE 13

### 2,3,4,9-Tetrahydro-1H-carbazole-6-carboxylic acid [Compound No. 13]

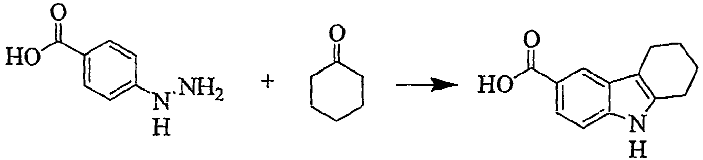

4-Hydrazinobenzoic acid (760 mg, 4.995 mmol) and cyclohexanone (637 mg, 6.490 mmol) were mixed and heated at 70°C for 15 minutes, and then sulphuric acid (20 mL, 10%) was added and the reaction mixture was heated under reflux, with stirring, for 30 minutes. The reaction mixture was cooled to room temperature and the solid formed was filtered off, washed with water and dried under reduced pressure to give the required product (1.010 g, 99%) as a light brown solid, mp275-278°C [Burtner, Lehmann, J.Amer.Chem.Soc., 1940, 62(3), 527-532, mp279°C]. IR (KBr): 3399, 2941, 2907, 2848, 1671, 1615, 1465, 1412, 1316, 1275, 1247, 1127, 954, 771 cm.^{-1 1}H NMR (DMSO-d₆): 12.23(1H, br), 11.01(1H, s), 8.01(1H, s), 7.64(1H, dd, J=1.6 & 8.4Hz), 7.29(1H, d, J=8.4Hz), 2.71(2H, t, J=5.0Hz), 2.65(2H, t, J=5.0Hz), 1.85-1.79(4H, m).

### EXAMPLE 14

### 1-Oxo-2,3,4,9-tetrahydro-1H-carbazole-6-carboxylic acid [Compound No. 14]

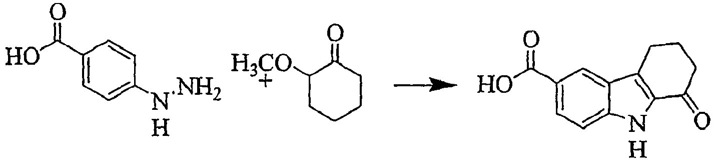

4-Hydrazinobenzoic acid (760 mg, 4.995 mmol) and 2-methoxycyclohexanone (960 mg, 7.493 mmol) were mixed together and heated at 80°C for 15minutes, and then sulphuric acid (30 mL, 10%) was added and the reaction mixture was heated under reflux for 30 minutes, with stirring. After cooling, the precipitated material was filtered, washed with water and n-hexane and dried under reduced pressure. The product was obtained as a brown solid (517 mg, 45%), mp275-280°C, although most of the material sublimed [S., Desikachari, P., Karnam, J. Rajend, Heterocycles, 1986, 24(3), 711-717, mp285-286°C]. Some of this material was further purified by column chromatography using silica gel and ethyl acetate/methanol 1/1. Fractions containing the product were collected, and the solvents were removed under reduced pressure to give a yellow solid. IR (KBr): 3248, 2931, 1680, 1650, 1612, 1417,1326, 1258, 1162, 901, 826, 770 cm.^{-1 1}H NMR (DMSO-d₆): 11.60(1H, s), 8.32(1H, s), 7.96(1H, d, J=8.6Hz), 7.34(1H, d, J=8.6Hz), 2.95(2H, t, J=6.4Hz), 2.55(2H, t, J=6.4Hz), 2.14(2H, t, J=6.4Hz).

### EXAMPLE 15

### 6-Methoxy-2,3,4,9-tetrahydro-1H-carbazole-2-carboxylic acid [Compound No. 15]

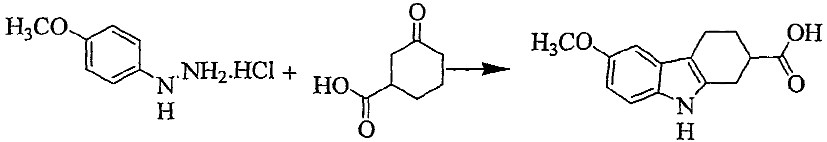

This material was prepared according to a standard literature procedure (80% yield) as white solid, R_{F}=0.22 (ethyl acetate/n-hexane 1/1), mp226-228°C [Allen, J.Heterocycl.Chem., 1970, 7, 239, mp226-227°C]. IR (KBr): 3386, 2926, 1695, 1591, 1481, 1457, 1432, 1287, 1243, 1214, 1133, 1029, 949, 809 cm.^{-1 1}H NMR (DMSO-d₆): 12.26(1H, br), 10.48(1H, s), 7.13(1H, d, J=6.6Hz), 6.82(1H, d, J=2.4Hz), 5.54(1H, dd, J=2.4 & 8.7Hz), 3.72(3H, s), 2.85-2.59(5H, m), 2.14(1H, m), 1.81(1H, m).

### EXAMPLE 16

### Methyl 2,3,4,9-tetrahydro-1H-carbazol-6-yl ether [Compound 16]

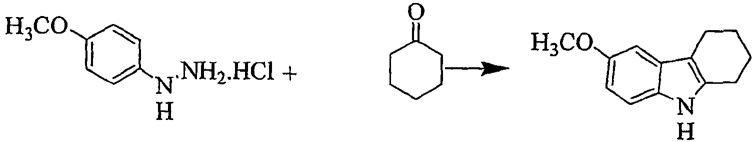

4-Methoxyhydrazine hydrochloride (1.746 g, 0.01 mol) was suspended in acetic acid (10 mL) and heated to 80°C, with stirring. Cyclohexanone (1.00 g, 0.01 mol) was added dropwise, with stirring. The reaction mixture was heated for 1 hour, cooled to room temperature and then kept in the fridge overnight. The colour of the reaction mixture changed to dark-brown. The solid material was filtered and washed with a small amount of acetic acid. More material was recovered from the filtrate. The combined solids were dried under reduced pressure to give an off-white crystalline material (1.596 g, 79%), mp108-110°C [Clark, D.W.; Jackson, A.H.; Prasitpan, N. and Shannon, P.V.R., J.Chem.Soc.Perkin Trans. II, 1982, 909-916, mp94-96°C], R_{F}=0.50 (ethyl acetate/n-hexane ¼). IR (KBr): 3389, 2915, 2845, 1622, 1589, 1482, 1434, 1218, 1134, 1028, 826, 798 cm.^{-1 1}H NMR (DMSO-d₆): 10.39(1H, s), 7.11(1H, d, J=8.7Hz), 6.81(1H, d, J=2.4Hz), 6.62(1H, dd, J=2.4 & 8.6Hz), 3.73(3H, s), 2.67(2H, t, J=5.4Hz), 2.57(1H, t, J=5.8Hz), 1.82-1.77(4H, m).

### EXAMPLE 17

### 7-Methoxy-1,2,3,4-tetrahydrocyclopenta[b]indole [Compound No.17]

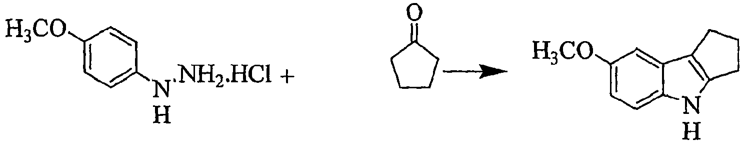

The procedure used was the same as that described in Example 16. The product was obtained as a brown solid (65% yield), mp128-130°C, R_{F}=0.60 (ethyl acetate/n-hexane ¼, alumina TLC plate. This material decomposed when applied to a silica gel TLC). IR (KBr): 3317, 2898, 2849, 1581, 1456, 1432, 1298, 1206, 1168, 1085, 1025, 846, 787 cm.^{-1 1}H NMR (DMSO-d₆): 10.45(1H, s), 7.21(1H, d, J=8.8Hz), 6.87(1H, d, J=2.4Hz), 6.67(1H, dd, J=2.5 & 8.7Hz), 3.78(3H, s), 2.85(2H, t, J=6.7Hz), 2.76(2H, t, J=6.7Hz), 2.48(2H, quintet, J=7.3Hz). HRFABMS: Found 188.10749 calculated for C₁₂H₁₄NO 188.10754.

### EXAMPLE 18

### 1,2,3,4-Tetrahydrocyclopenta[b]indole-7-carboxylic acid [Compound No.18]

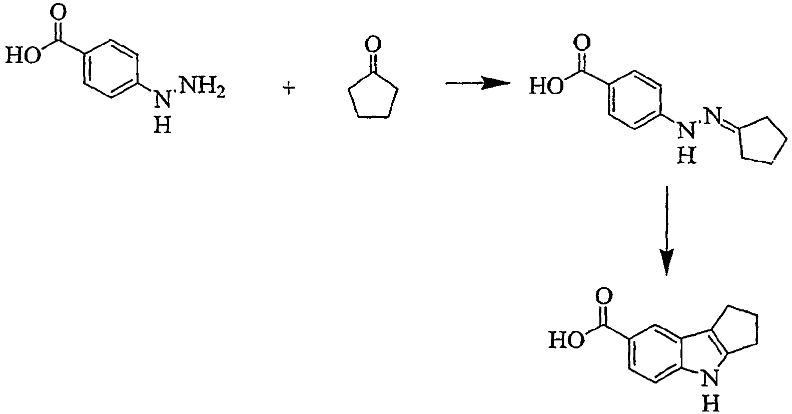

4-Hydrazinobenzoic acid (637 mg, 4.187 mmol) was suspended in acetic acid (10 mL) and heated to 50°C. Cyclopentanone (352 mg, 4.187 mmol) was added dropwise, with stirring, to the reaction mixture, which was then heated to 110°C for 1 hour. A clear light brown solution was formed during the course of the reaction. The reaction mixture was then cooled to room temperature and the yellow solid which precipitated was filtered and washed with a small amount of acetic acid (dilute) and water and was then dried under reduced pressure at 45°C to give 4-(2-cyclopentylidene-hydrazino)benzoic acid (711 mg, 78%), mp 250-253°C. The hydrazone intermediate (300 mg, mmol) was suspended in sulphuric acid (3 mL, 10%) and heated under reflux for 15 minutes. The solid lilac material that formed was filtered after the solution had been cooled to room temperature, washed with water and dried under reduced pressure (30 mg, 11%), mp270-274°C (most of the material sublimed and the melting point recorded was that of the sublimed crystals). IR (KBr): 3380, 2946, 1656, 1616, 1473, 1410, 1347, 1295, 1261, 1129, 770, 746 cm.^{-1 1}H NMR (DMSO-d₆): 11.17(1H, s), 7.98(1H, s), 7.62(1H, dd, J=1.6 & 8.5Hz), 7.32(1H, d, J=8.5Hz), 2.83(2H, t, J=7.5Hz), 2.77(2H, t, J=6.8Hz), 2.47(2H, quintet, J=7.2Hz). HRFABMS: Found 202.08733 calculated for C₁₂H₁₂NO₂ 202.08680.

### EXAMPLE 19

### 5,8-Dimethyl-2,3,4,9-tetrahydro-1H-carbazole-2-carboxylic acid [Compound No. 19]

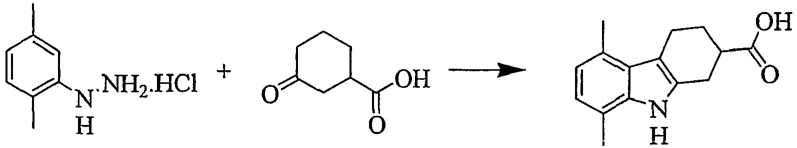

N-(2,5-Dimethylphenyl)hydrazine hydrochloride (243 mg, 1.407 mmol) was dissolved in acetic acid (3 mL) to which 3-oxocyclohexanecarboxylic acid (200 mg, 1.407 mol) dissolved in acetic acid (2 mL) was added, with stirring. The reaction mixture was heated under reflux for 2 hours. The cooled reaction mixture was diluted with brine and extracted with ethyl acetate. The organic layer was dried (MgSO₄) and the solvent was removed under reduced pressure. The crude product was applied to a silica gel chromatography column using ethyl acetate/n-hexane (¼) as eluant (R_{F}=0.50, ethyl acetate/n-hexane 1/1). The product was obtained as a yellow solid (153 mg, 45%), mp228-230°C. IR (KBr): 3390, 2928, 1702, 1436, 1294, 1230, 946, 800 cm.^{-1 1}H NMR 12.21(1H, s), 10.46(1H, s), 6.63(1H, d, J=7.2Hz), 6.54(1H, d, J=7.2Hz), 3.01-2.74(5H, m), 2.50(3H, s), 2.34(3H, s), 2.13(1H, m), 1.81(1H, m). HRFABMS: Found 244.13394 calculated for C₁₅H₁₈NO₂ 244.13375.

### EXAMPLE 20

### (4E)-1,2,3,9-Tetrahydro-4H-carbazol-4-one oxime [Compound No. 20]

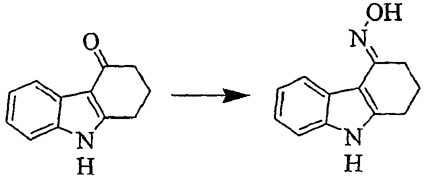

A mixture of 1,2,3,4-tetrahydrocarbazole-4-one (450 mg, 2.432 mmol) hydroxylamine hydrochloride (253 mg, 3.640 mmol, 1.5 molar excess), sodium acetate (298 mg, 3.640 mmol, 1.5 molar excess), ethanol (5 mL) and water (2 mL) was heated under reflux in a nitrogen atmosphere for 4 hours. The cooled mixture was concentrated under reduced pressure and the residue was suspended in water. The crystalline material was collected by filtration, washed with water and dried under reduced pressure to yield (410 mg, 84%), mp200-205°C (decomposition), of the crude product. This material was chromatographed on silica gel with ethyl acetate/n-hexane 1/3 (R_{F}=0.35). The product was obtained as a white crystalline material (380 mg, 78%), mp206-208°C (decomposition), (Hester, J.B., J.Org.Chem., 1967, 32, 3804-3807, mp208.5-210°C). IR (KBr): 3415, 2925, 1620, 1556, 1481, 1451, 1418, 920, 890, 853, 746 cm.^{-1 1}H NMR (DMSO-d₆): 1.19(1H, s), 10.21(1H, s), 7.89(1H, d, J=7.6Hz), 7.31(1H, d, J=7.6Hz), 7.08-5.99(2H, m), 2.79(2H, t, J=6.1Hz), 2.67(2H, t, J=6.1Hz), 1.91(2H, quintet, J=6.2Hz).

### EXAMPLE 21

### (3E)-1,2,4,9-Tetrahydro-3H-carbazol-3-one oxime [Compound No. 21]

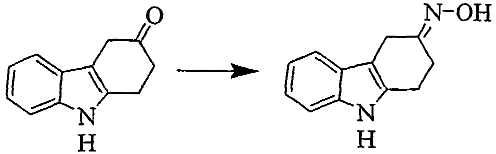

The procedure used was the same as that described in Example 20. The required product was obtained as a light brown solid (20 mg, 22%), mp173-175°C (decomposition), R_{F}=0.35 (ethyl acetate/n-hexane 1/3). IR (KBr): 3392, 3280, 1461, 14, 39, 1356, 1327, 1224, 1004, 920, 741 cm.^{-1 1}H NMR (DMSO-d₆): 10.76(1H, s), 10.52(1H, s), 7.39(1H, d, J=7.7Hz), 7.27(1H, d, J=7.7Hz), 7.02(1H, t, J=7.0Hz), 5.94(1H, t, J=7.0Hz), 3.50(2H, s), 2.87(2H, t, J=6.5Hz), 2.60(2H, t, J=6.5Hz). HREIMS: Found 200.09506 calculated for C₁₂H₁₂N₂O 200.09496.

### EXAMPLE 22

### Synthesis of Compound No. 22

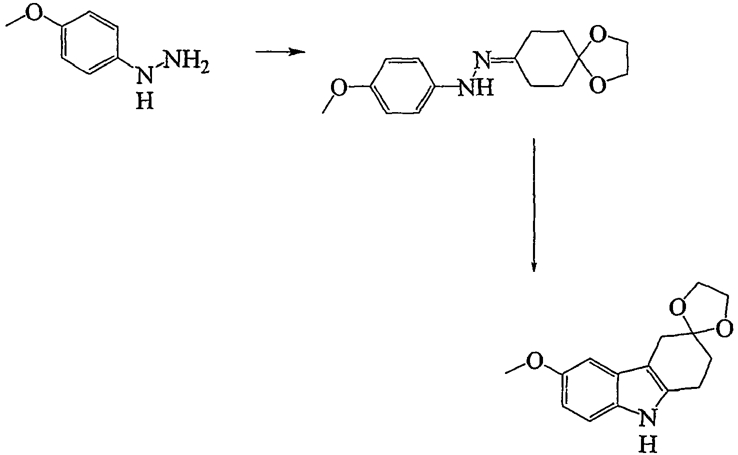

4-Methoxyhydrazine hydrochloride (1.172 g, 6.712 mmol) was dissolved in water (25 mL) to which saturated sodium hydrogen carbonate was added until the solution was basic and effervescence ceased. Dichloromethane was added and the organic layers were collected after extraction. Drying (MgSO₄), followed by removal of the solvent, provided the required material as a pale yellow crystalline material, which was used in the next step without further purification (0.710 g) The ketone (806 mg, 5.160 mmol) was added to 4-methoxyhydrazine, followed by toluene (50 mL). The reaction mixture was heated under reflux for 30 minutes, and then the solvent was removed under reduced pressure to give the product, 1,4-dioxaspiro[4.5]decan-8-one (4-methoxyphenyl)hydrazone, as a brown oil, which was used in the next step without further purification. Ethylene glycol (20 mL) was added and the reaction mixture was heated at 180°C for 3 hours under nitrogen. The cooled solution was poured over ice water and extracted with dichloromethane. The organic layers were collected and dried (MgSO₄), and the solvent was removed under reduced pressure to give a dark brown oil. Purification by silica gel column chromatography and eluting with ethyl acetate/n-hexane (1/3) gave the required product as a pale yellow microcrystalline material, after recrystallisation from ethyl acetate/n-hexane (630 mg, 36%), mp168-169°C. IR (KBr): 3345, 2891, 1627, 1597, 1483, 1456, 1326, 1212, 1139, 1120, 1101, 1062, 1031, 951 cm.^{-1 1}H NMR (DMSO-d₆): 10.50(1H, s), 7.13(1H, d, J=8.7Hz), 6.81(1H, d, J=2.8Hz), 6.64(1H, dd, J=2.5 & 8.7Hz), 3.95(2H, s), 3.73(3H, s), 2.81(4H, s & t), 1.94(2H, t, J=6.7Hz). HREIMS: Found 259.12065 calculated for C₁₅H₁₇NO₃ 259.12084.

### EXAMPLE 23

### 6-Methoxy-1,2,4,9-tetrahydro-3H-carbazol-3-one [AIK-18/49]

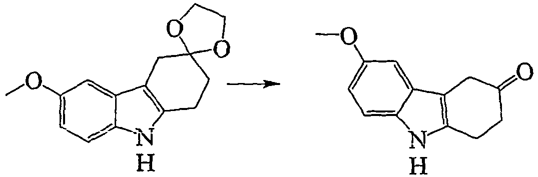

The starting material (375 mg, 1.446 mmol, prepared as described in Example 22) was dissolved in tetrahydrofuran (10 mL) and hydrochloric acid (10 mL, 50%), with stirring, at room temperature. The reaction mixture was heated between 40-50°C for 4 hours. Sodium carbonate (aqueous, saturated) was added dropwise to the cooled solution, with stirring, until effervescence ceased. Dichloromethane was then added and the reaction mixture was extracted. The organic layer was collected and dried (MgSO₄), and the solvent was removed under reduced pressure. The crude product was applied to a chromatography column and eluted with ethyl acetate/n-hexane (1/2). The product was recrystallised from ethyl acetate/n-hexane (R_{F}=0.50) to give the pure material as a white microcrystalline material (212 mg, 68%), mp155-158°C (Caubere, C.; Caubere, P.; Renard, P.; Bizot-Espiart, J.; Jamart-Gregoire, B.; Tetrahedron Lett. 1993, 34(43), 6889-6892, mp149-151°C). IR (KBr): 3275, 2951, 2898, 1690, 1603, 1486, 1209, 1136, 1020, 827 cm.^{-1 1}H NMR (DMSO-d₆): 10.73(1H, s), 7.19(1H, d, J=8.7Hz) 6.86(1H, d, J=2.3Hz), 6.69(1H, dd, J=2.5 & 8.7Hz), 3.73(3H, s), 3.47(2H, s), 3.08(2H, t, J=6.9Hz), 2.69(2H, t, J=6.9Hz).

### EXAMPLE 24

### Protocol

Organotypic hippocampal slice cultures were prepared using the basic method of Pringle et al [Brain Res. 755, 36-46 (1997)] modified as follows:

Wistar rat pups (8-11 days old) were decapitated and the hippocampus rapidly dissected into ice-cold Gey's balanced salt solution supplemented with 4.5mg/ml glucose. Slices were separated and plated onto Millicell CM culture inserts (4 per well) and maintained at 37°C/5% CO₂ for 14 days. Maintenance medium consisted of 25% heat-inactivated horse serum, 25% Hank's balanced salt solution (HBSS) and 50% minimum essential medium with added Earle's salts (MEM) supplemented with 1mM glutamine and 4.5mg/ml glucose. Medium was changed every 3-4 days.

Experimental hypoxia was performed as described previously (Pringle et al., Stroke 27, 21-24 (1996)& Brain Res. 755, 36-46 (1997)]. Briefly, cultures were transferred to serum free medium (SFM - 75% MEM, 25% HBSS supplemented with 1mM glutamine and 4.5mg/ml glucose) containing 5 µg/ml of the fluorescent exclusion dye propidium iodide (PI). Cultures were allowed to equilibrate in SFM for 60 minutes prior to imaging. PI fluorescence was detected using a Leica DMIL inverted microscope fitted with a rhodamine filter set. Any cultures in which PI fluorescence was detected at this stage were excluded from further study. Hypoxia was induced by transferring cultures to SFM (+PI) which had been saturated with 95%N₂/5%CO₂. Culture plates (without lids) were then sealed into an airtight chamber in which the atmosphere was saturated with 95%N₂/5%CO₂ by continuously blowing through gas at 10 L/minute for ten minutes before being sealed and placed in the incubator for 170 minutes (total time of hypoxia was therefore 180 minutes). At the end of the hypoxic period cultures were returned to normoxic SFM containing PI and placed back in the incubator for 24 hours.

Neuronal damage was assessed using ImageJ running on a PC. Images were captured using a CCD camera and saved for offline analysis. Light transmission images were captured prior to the addition of drugs, and PI fluorescence images recorded at the end of the 24-hour post-hypoxia recovery period. The area of the CA1 sub-region was determined from the transmission image. The area of PI fluorescence in the CA1 was measured using the threshold function on ImageJ, and neuronal damage expressed as the percentage of the CA1 in which PI fluorescence was detected above background.

Unpaired Student's t-tests were employed to ascertain statistical significance.

A series of compounds were examined for possible neuroprotection against hypoxia in organotypic hippocampal slice cultures. Compounds No. 4, 6, 7, 8, 10, 14, 15, 16, 17, 20, 21, 22 and 23 were dissolved in DMSO to a concentration of 1 mg/ml.

7β-hydroxy-epiandrosterone (7β-OH EPIA), a neuroprotective compound, at 100nM concentrations, was employed as a positive control. A stock solution of 1 mg/ml was dissolved in ethanol. Final dilutions were made in SFM.

The efficacy of all compounds was assessed using a pre-during and post-hypoxia paradigm - compounds were present in the medium 45 minutes pre-hypoxia, the 3 hours of hypoxia and 24 hours post-hypoxia.

The results are given in the following Table 2, which shows the % damage to the neurons in the hypoxia model when compared against the control (hypoxia only - 100% damage). The lower the % damage, the more neuroprotective the compound. The last column in the table shows the % damage with 7β-OH EPIA, (positive control). All the values are statistically significant.

**Table 2**

| Compound No. | % damage in hypoxia model with compound | % damage with positive control |
|---|---|---|
| 4 | 58% | 57% |
| 6 | 63% | 50% |
| 7 | 70% | 50% |
| 8 | 59% | 50% |
| 10 | 76% | 51% |
| 14 | 53% | 34% |
| 15 | 65% | 34% |
| 16 | 60% | 43% |
| 17 | 35% | 33% |
| 20 | 54% | 55% |
| 21 | 40% | 55% |
| 22 | 75% | 55% |
| 23 | 57% | 55% |

## Claims

1. The use of compounds of formula (I): ' 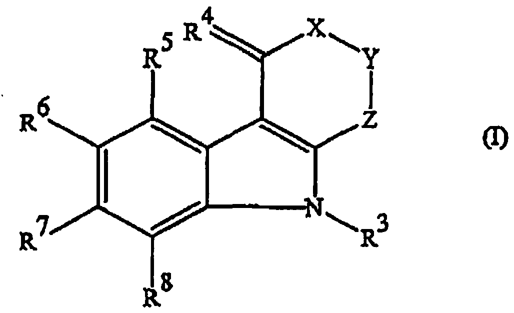 in which:
X represents a group of formula >CR¹R² or, when R⁶ does not represent a hydrogen atom, a group of formula >SO₂;
Y represents a group of formula >CR¹R²;
Z represents a group of formula >C=O, a group of formula >CH₂ or a direct bond;
R¹ represents a hydrogen atom and R² represents a hydrogen atom, a carboxy group or
a hydroxy group;
or
R¹ and R² together represent an oxo group, an ethylenedioxy group or a hydroxyimino group;
R³ represents a hydrogen atom or a straight or branched C₁ - C₁₀ alkyl group;
R⁴ represents two hydrogen atoms, or an oxo or hydroxyimino group;
R⁵ represents a hydrogen atom, a straight or branched C₁ - C₁₀ alkyl group or a halogen atom;
R⁶ represents a hydrogen atom, a straight or branched C₁-C₁₀ alkoxy group or a carboxy group;
R⁷ and R⁸ are the same as or different from each other and each represents a hydrogen atom, a straight or branched C₁-C₁₀ alkyl group or a halogen atom;
and pharmaceutically acceptable salts thereof for the manufacture of a medicament for protection against ischaemic damage to tissues of peripheral organs, which are selected from any functional tissue in the boby except the brain and the spiral cord.

2. The use according to Claim 1, in which:
X represents a group of formula >CR¹R² where R¹ represents a hydrogen atom and R² represents a hydrogen atom, a hydroxy group or a carboxy groups, or R¹ and R² together represent an oxo group or an ethylenedioxy group;
Y represents a group of formula >CR¹R² where R¹ represents a hydrogen atom and R² represents a hydrogen atom or a carboxy group;
R³ represents a hydrogen atom;
R⁴ represents two hydrogen atoms or an oxo group;
R⁵ represents a hydrogen atom;
R⁶ represents a hydrogen atom, a C₁ - C₄ alkoxy group or a carboxy group; and R⁷ and R⁸ are the same as or different from each other and each represents a hydrogen atom or a C₁ - C₄ alkyl group;
and salts thereof.

3. The use according to Claim 1, in which said compound is a compound of formula:

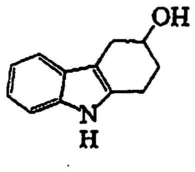

4. The use according to Claim 1, in which said compound is a compound of formula:

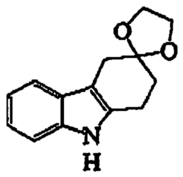

5. The use according to Claim 1, in which said compound is a compound of formula:

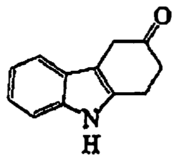

6. The use according to Claim 1, in which said compound is a compound of formula:

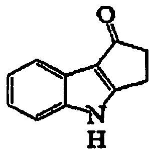

7. The use according to Claim 1, in which said compound is a compound of formula:

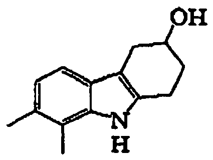

8. The use according to Claim 1, in which said compound is a compound of formula:

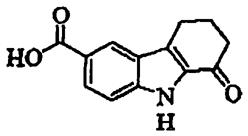

9. The use according to Claim 1, in which said compound is a compound of formula:

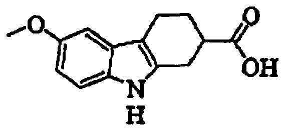

10. The use according to Claim 1, in which said compound is a compound of formula:

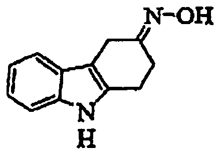

11. The compound of formula:

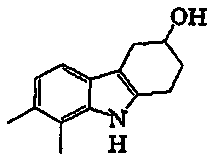

12. The compound of formula:

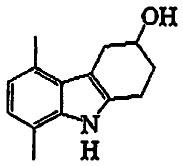

13. The compound of formula:

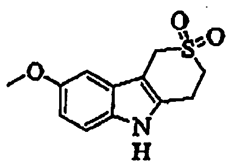

14. The compound of formula: 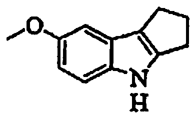 for use as a medicament.

15. The compound of formula:

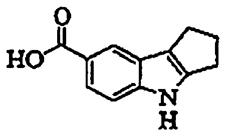

16. for use as a medicament. 16. The compound of formula:

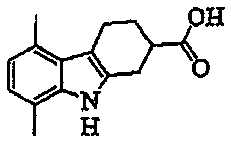

17. The compound of formula: 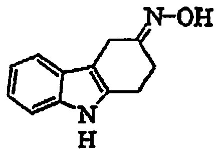 for use as a medicament.

18. The compound of formula: 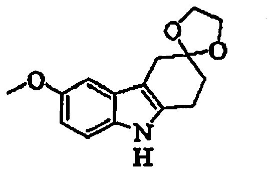 for use as a medicament.

## Patentansprüche

1. Verwendung von Verbindungen der Formel (I): 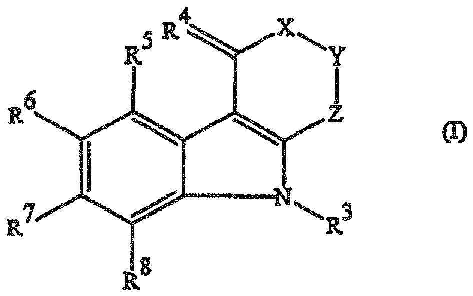 worin:
X eine Gruppe der Formel >CR¹R² oder, wenn R⁶ nicht ein Wasserstoffatom darstellt, eine Gruppe der Formel >SO₂ darstellt;
Y eine Gruppe der Formel >CR¹R² darstellt;
Z eine Gruppe der Formel >C=O, eine Gruppe der Formel >CH₂ oder eine direkte Bindung darstellt;
R¹ ein Wasserstoffatom darstellt und R² ein Wasserstoffatom, eine Carboxygruppe oder eine Hydroxygruppe darstellt;
oder
R¹ und R² zusammen eine Oxogruppe, eine Ethylendioxygruppe oder eine Hydroxyiminogruppe darstellen;
R³ ein Wasserstoffatom oder eine geradkettige oder verzweigte C₁-C₁₀-Alkylgruppe darstellt;
R⁴ zwei Wasserstoffatome oder eine Oxo- oder Hydroxyiminogruppe darstellt;
R⁵ ein Wasserstoffatom, eine geradkettige oder verzweigte C₁-C₁₀-Alkylgruppe oder ein Halogenatom darstellt;
R⁶ ein Wasserstoffatom, eine geradkettige oder verzweigte C₁-C₁₀-Alkoxygruppe oder eine Carboxygruppe darstellt;
R⁷ und R⁸ gleich oder verschieden voneinander sind und jeweils ein Wasserstoffatom, eine geradkettige oder verzweigte C₁-C₁₀-Alkylgruppe oder ein Halogenatom darstellen;
und pharmazeutisch annehmbaren Salzen davon für die Herstellung eines Medikaments zum Schutz vor ischämischem Schaden an Geweben der peripheren Organe, die ausgewählt sind aus jedem funktionellen Gewebe im Körper, mit Ausnahme des Gehirns und des Rückenmarks.

2. Verwendung gemäß Anspruch 1, wobei:
X eine Gruppe der Formel >CR¹R² darstellt, worin R¹ ein Wasserstoffatom darstellt und R² ein Wasserstoffatom, eine Hydroxygruppe oder eine Carboxygruppe darstellt, oder R¹ und R² gemeinsam eine Oxogruppe oder eine Ethylendioxygruppe darstellen;
Y eine Gruppe der Formel >CR¹R² darstellt, worin R¹ ein Wasserstoffatom darstellt und R² ein Wasserstoffatom oder eine Carboxygruppe darstellt;
R³ ein Wasserstoffatom darstellt;
R⁴ zwei Wasserstoffatome oder eine Oxogruppe darstellt;
R⁵ ein Wasserstoffatom darstellt;
R⁶ ein Wasserstoffatom, eine C₁-C₄-Alkoxygruppe oder eine Carboxygruppe darstellt; und R⁷ und R⁸ gleich oder verschieden voneinander sind und jeweils ein Wasserstoffatom oder eine C₁-C₄-Alkylgruppe darstellen;
und Salzen davon.

3. Verwendung gemäß Anspruch 1, wobei die genannte Verbindung eine Verbindung der Formel ist:

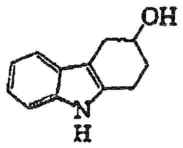

4. Verwendung gemäß Anspruch 1, wobei die genannte Verbindung eine Verbindung der Formel ist:

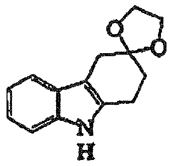

5. Verwendung gemäß Anspruch 1, wobei die genannte Verbindung eine Verbindung der Formel ist:

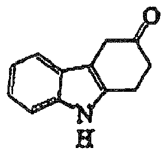

6. Verwendung gemäß Anspruch 1, wobei die genannte Verbindung eine Verbindung der Formel ist:

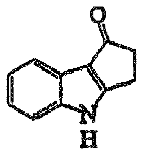

7. Verwendung gemäß Anspruch 1, wobei die genannte Verbindung eine Verbindung der Formel ist:

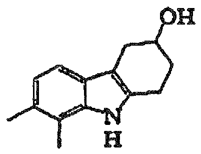

8. Verwendung gemäß Anspruch 1, wobei die genannte Verbindung eine Verbindung der Formel ist:

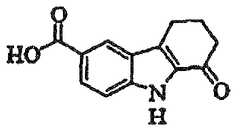

9. Verwendung gemäß Anspruch 1, wobei die genannte Verbindung eine Verbindung der Formel ist:

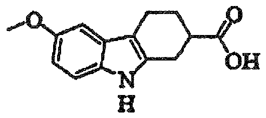

10. Verwendung gemäß Anspruch 1, wobei die genannte Verbindung eine Verbindung der Formel ist:

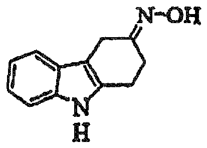

11. Verbindung der Formel:

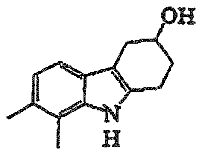

12. Verbindung der Formel:

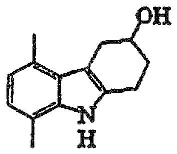

13. Verbindung der Formel:

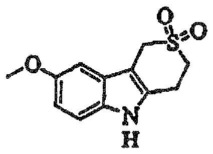

14. Verbindung der Formel: 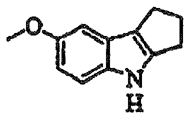 zur Verwendung als ein Medikament.

15. Verbindung der Formel: 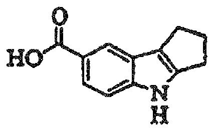 zur Verwendung als ein Medikament.

16. Verbindung der Formel:

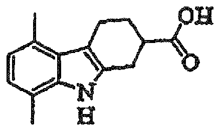

17. Verbindung der Formel: 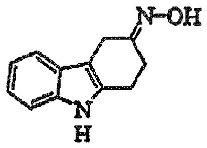 zur Verwendung als ein Medikament.

18. Verbindung der Formel: 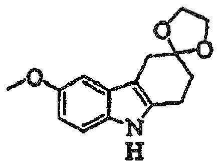 zur Verwendung als ein Medikament.

## Revendications

1. Utilisation de composés de formule (I) : 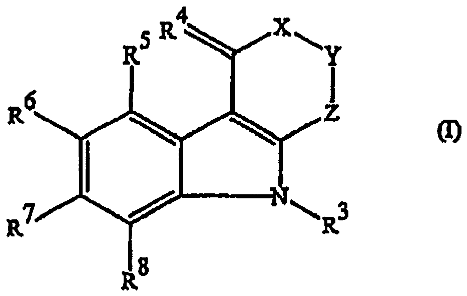 dans laquelle :
X représente un groupe de formule >CR¹R² ou, lorsque R⁶ ne représente pas un atome d'hydrogène, un groupe de formule >SO₂ ;
Y représente un groupe de formule >CR¹R² ;
Z représente un groupe de formule >C=O, un groupe de formule >CH₂ ou une liaison directe ;
R¹ représente un atome d'hydrogène et R² représente un atome d'hydrogène, un groupe carboxy ou un groupe hydroxy ;
ou
R¹ et R² représentent conjointement un groupe oxo, un groupe éthylènedioxy ou un groupe hydroxyimino ;
R³ représente un atome d'hydrogène ou un groupe alkyle en C₁ à C₁₀ linéaire ou ramifié ;
R⁴ représente deux atomes d'hydrogène, ou un groupe oxo ou hydroxyimino ;
R⁵ représente un atome d'hydrogène, un groupe alkyle en C₁ à C₁₀ linéaire ou ramifié ou un atome d'halogène ;
R⁶ représente un atome d'hydrogène, un groupe alcoxy en C₁ à C₁₀ linéaire ou ramifié ou un groupe carboxy ;
R⁷ et R⁸ sont identiques ou différents l'un de l'autre et représentent chacun un atome d'hydrogène, un groupe alkyle en C₁ à C₁₀ linéaire ou ramifié ou un atome d'halogène ;
et des sels pharmaceutiquement acceptables de ceux-ci pour la fabrication d'un médicament destiné à la protection contre un endommagement ischémique de tissus d'organes périphériques, qui sont sélectionnés parmi tout tissu fonctionnel dans le corps à l'exception du cerveau et de la moelle épinière.

2. Utilisation selon la revendication 1, dans laquelle :
X représente un groupe de formule >CR¹R² où R¹ représente un atome d'hydrogène et R² représente un atome d'hydrogène, un groupe hydroxy ou un groupe carboxy, ou R¹ et R² représentent conjointement un groupe oxo ou un groupe éthylènedioxy ;
Y représente un groupe de formule > CR¹R² où R¹ représente un atome d'hydrogène et R² représente un atome d'hydrogène ou un groupe carboxy ;
R³ représente un atome d'hydrogène ;
R⁴ représente deux atomes d'hydrogène ou un groupe oxo ;
R⁵ représente un atome d'hydrogène ;
R⁶ représente un atome d'hydrogène, un groupe alcoxy en C₁ à C₄ ou un groupe carboxy ; et R⁷ et R⁸ sont identiques ou différents l'un de l'autre et représentent chacun un atome d'hydrogène ou un groupe alkyle en C₁ à C₄ ;
et des sels de ceux-ci.

3. Utilisation selon la revendication 1, dans laquelle ledit composé est un composé de formule :

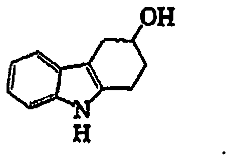

4. Utilisation selon la revendication 1, dans laquelle ledit composé est un composé de formule :

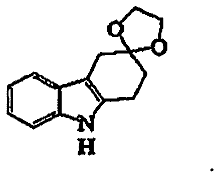

5. Utilisation selon la revendication 1, dans laquelle ledit composé est un composé de formule :

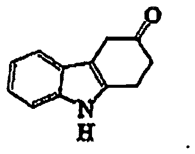

6. Utilisation selon la revendication 1, dans laquelle ledit composé est un composé de formule :

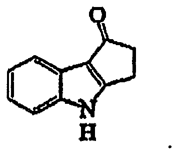

7. Utilisation selon la revendication 1, dans laquelle ledit composé est un composé de formule :

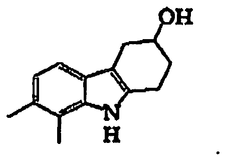

8. Utilisation selon la revendication 1, dans laquelle ledit composé est un composé de formule :

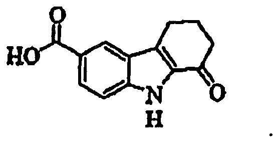

9. Utilisation selon la revendication 1, dans laquelle ledit composé est un composé de formule :

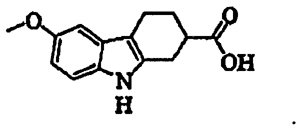

10. Utilisation selon la revendication 1, dans laquelle ledit composé est un composé de formule :

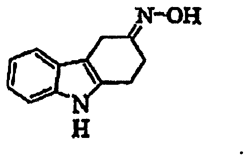

11. Composé de formule :

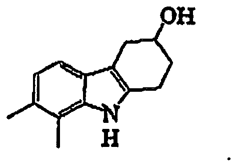

12. Composé de formule :

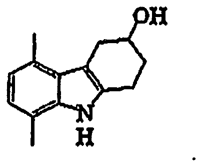

13. Composé de formule :

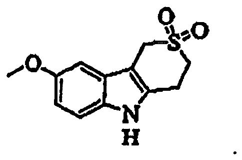

14. Composé de formule : 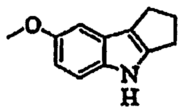 pour une utilisation en tant que médicament.

15. Composé de formule : 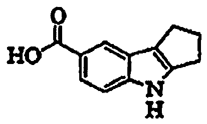 pour une utilisation en tant que médicament.

16. Composé de formule :

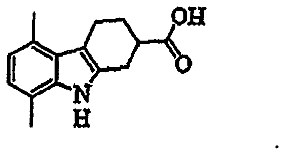

17. Composé de formule : 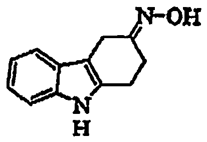 pour une utilisation en tant que médicament.

18. Composé de formule : 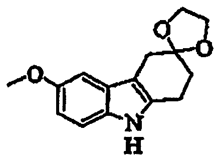 pour une utilisation en tant que médicament.
